# EUROPÄISCHE PATENTSCHRIFT

(11) **EP 2 578 268 B1**
(45) Veröffentlichungstag und Bekanntmachung des Hinweises auf die Patenterteilung: **22.07.2020**
(21) Anmeldenummer: 12183871.8
(22) Anmeldetag: 11.09.2012
(51) Int. Cl.: A61N 1/37

(54) **Temperatursensor für ein implantierbares medizinisches Gerät**
Temperature sensor for an implantable medical device
Capteur de température pour un appareil médical implantable

(30) Priorität: 06.10.2011 US 201161543818 P
(43) Veröffentlichungstag der Anmeldung: 10.04.2013
(73) Patentinhaber: BIOTRONIK SE & Co. KG, 12359 Berlin (DE)
(72) Erfinder: Dörr, Thomas, 12437 Berlin (DE); Weiss, Ingo, 12435 Berlin (DE)
(74) Vertreter: Galander, Marcus

(56) Entgegenhaltungen:
- US-A1- 2010 069 921
- US-A1- 2011 066 028
- US-A1- 2011 160 791
- US-B1- 6 681 135

## Beschreibung

Die Erfindung betrifft einen Sensor für Temperaturänderungen an Elektrodenpolen, insbesondere von Elektrodenpolen an einer implantierbaren Stimulationselektrodenleitung für einen Herzstimulator, implantierbaren Impulsgenerator (IPG) Kardioverter/Defibrillator (ICD) oder dergleichen.

Derartige implantierbare medizinische Geräte sind beispielsweise implantierbare Defibrillatoren (ICDs) oder Herzschrittmacher, die über Elektrodenleitungen elektrische Stimulationsimpulse an Herzgewebe (Myocard) abgeben können oder über entsprechende Sensoren elektrische Potentiale im Herzgewebe erfassen können. Andere Implantate, wie z.B. Neurostimulatoren, dienen der Stimulation anderen Gewebes. Im Zusammenhang mit Herzschrittmachern ist es bekannt, dass diese selbsttätig automatische Nachsorgen, z.B. als sog. Chronjobs, d.h. hinsichtlich des Ablaufs und des Zeitpunkts vorprogrammierte Selbsttests, durchführen, bei denen, wie nach heutigen Richtlinien üblich, bestimmte Parameter, so z.B. Reizschwelle, Elektrodenimpedanz, Batteriespannung, Signalamplituden etc., erfasst werden. Hierzu dient die von der Steuereinheit angesteuerte Testeinheit.

Implantierbare Herzschrittmacher oder Defibrillatoren sind typischerweise mit Elektrodenleitungen für die Elektrostimulation verbunden, die den Nachteil mit sich bringen, dass sich ihr elektrischer Leiter in einem Kernspintomografen (auch als Magnetresonanztomograf bezeichnet) erwärmen kann, weil die im Kernspintomografen herrschenden wechselnden Magnetfelder in dem elektrischen Leiter nicht unbeachtliche elektrische Ströme induzieren. Auch können solche induzierten Ströme über Elektrodenpole der Elektrodenleitung an umliegendes Gewebe abgegeben werden und so beispielsweise zu unerwünschten Gewebeerwärmungen führen.

An implantierbaren Herzschrittmachern oder Defibrillatoren (im Folgenden auch gemeinsam als Herzstimulatoren oder IPG (implantable pulse generator) bezeichnet) ist nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens und/oder zum Abfühlen elektrischer Felder, um im Rahmen des sogenannten Sensings eine Aktivität eines Herzens abfühlen zu können. Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tippelektrode am distalen Ende der Elektrodenleitung vorgesehen. Die Elektrodenpole sind über eine oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalem Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden.

Andererseits können die Elektrodenpole auch Wärme infolge von in die Elektrodenleitung induzierter Ströme an die umliegende Körperflüssigkeit oder das umliegende Gewebe abgeben.

Es existieren bereits verschiedene Lösungen zur Reduktion der MRT-induzierten Elektrodenerwärmungen, überwiegend aber mit modifizierten Elektroden. Der oft erforderliche Temperatursensor wird dabei als vorhanden betrachtet. So offenbaren die US2011/066028 und die US2011/0160791 einen Schrittmacher mit einer Impedanzmessung und einen Temperatursensor. Zudem ist aus der US2010/0069921 ein System zur Bewertung der Läsionsbildung in Gewebe bekannt, das einen Temperatursensor in Verbindung mit einer Impedanzerfassungseinrichtung aufweist.

Derzeit ist die Anwendung einer MRT-Untersuchung bei Patienten mit einem IPG kontraindiziert. Die in der Umgebung des MRT auftretenden Probleme sind vor allem die Erwärmung der Elektrodenspitze durch die starken elektromagnetischen Wechselfelder im MRT.

Lösungsansätze betreffen überwiegend die Modifikation der Elektrode, so z.B. ist der Einsatz von Bandpassfiltern in der Elektrode beschrieben. Nachteile einer solchen Lösung sind neben dem konstruktiven Aufwand vor allem die Unterbrechung der therapieführenden Elektrodenleitung mit Widerständen, induktiven und kapazitiven Bauelementen und damit verbunden eine mögliche Beeinträchtigung der Therapiefunktion, wie z.B. die Abgabe hochfrequenter Signale (Hemodynamischersensor, kurz HDS- oder Closed Loop- Stimulation kurz CLS-Strompulse mit steilen Flanken etc.). Außerdem wird die Zuverlässigkeit der durch die Bandsperre unterbrochenen Elektrodenleitung durch die zusätzlichen Fügestellen herabgesetzt und damit eine potenzielle Fehlerquelle für den Ausfall einer solchen Elektrode geschaffen.

Nachteil aller dieser Lösungen ist die Notwendigkeit dieser Spezialelektrode, mit ggf. ungünstigeren Implantations- und Langzeiteigenschaften. Ebenso können Patienten mit liegenden Standardelektroden (MR unsicher) nicht MR-tauglich nachgerüstet werden, da auch diese Spezialelektroden die Anwesenheit von Standardelektroden als Ausschlusskriterium ausweisen. Selbst Lösungen, die im Implantat realisiert wären, erfordern einen Sensor, der problemspezifisch ein Temperatursensor an der Elektrodenspitze ist. Einen solchen zu integrieren bedeutet ebenso, eine Spezialelektrode zu konstruieren.

Viele Ansätze, z.B. zur Reduktion der Erwärmung an Elektrodenpolen während einer Kernspintomographie (Magnetresonaztomographie MRI), erfordern einen Temperatursensor, um eine solche Erwärmung festzustellen.

Der Erfindung liegt die Aufgabe zugrunde, eine sichere MRT-Untersuchung von Patienten mit elektronischen Implantaten zu realisieren.

Erfindungsgemäß wird diese Aufgabe durch einen Temperatursensor zum Erfassen einer Erwärmung wenigstens eines Elektrodenpols einer temporär oder dauerhaft implantierbaren Einrichtung, wie beispielsweise einer Elektrodenleitung mit einem längsgestreckten elektrischen Leiter, gelöst, wobei der Temperatursensor eine Impedanzerfassungseinrichtung aufweist oder mit einer solchen verbunden ist und der zur Auswertung einer von der Impedanzerfassungseinrichtung erfassten Elektrodenpolimpedanz derart ausgebildet ist, dass die Auswertung hinsichtlich eines temperaturabhängigen Merkmals der Elektrodenpolimpedanz erfolgt. Hierzu ist die Impedanzerfassungseinrichtung mit dem wenigstens einen Elektrodenpol elektrisch verbunden oder ausgebildet und angeordnet, mit dem wenigstens einen Elektrodenpol elektrisch verbunden zu werden. Zudem ist der Temperatursensor oder die Impedanzerfassungseinrichtung dazu ausgebildet, eine Elektrodenpolimpedanz nur innerhalb wenigstens eines ereignisabhängigen Zeitfensters zu erfassen und auszuwerten.

Der erfindungsgemäße Temperatursensor erlaubt es, dass die Temperaturerfassung mit einer Standardelektrode erfolgen kann, weil sich die Messvorrichtung außerhalb der Elektrodenleitung, z.B. im Implantat, befinden kann und über die üblichen Kontakte am proximalen Ende einer Elektrodenleitung mit der Elektrodenpolen der Elektrodenleitung elektrisch verbunden werden kann. Die Erfindung ermöglicht es somit, ohne Einsatz einer Spezialelektrode (d.h. mit eingebautem Temperaturmesselement) eine Temperaturmessung nur mit Messmitteln im Implantat durchzuführen.

Vorzugsweise ist der Temperatursensor Bestandteil eines implantierbaren medizinischen Gerätes, an das wenigstens eine Elektrodenleitung mit wenigstens einem Elektrodenpol anschließbar oder angeschlossen ist; d.h. ein Erfindungsaspekt betrifft ein implantierbares medizinisches Gerät - kurz: ein Implantat - mit einem erfindungsgemäßen Temperatursensor.

Vorzugsweise ist ein solches implantierbares medizinisches Gerät mit erfindungsgemäßem Temperatursensor zusätzlich mit einer Stellvorrichtung ausgerüstet, die in Abhängigkeit des temperaturabhängigen Merkmals der Elektrodenpolimpedanz eine Implantatseingangsschaltung hinsichtlich der Elektrodenabschlussimpedanz so parametriert, dass eine Erwärmung an dem jeweiligen Elektrodenpol minimiert wird.

Dies ermöglich ein Steuerverfahren, mit dem die Erwärmung der Elektrodenspitze im Kernspintomografen (Magnetresonanztomografen, MRT) reduziert werden kann. Dieses Steuerverfahren basiert auf einem Ansatz, die Eingangsimpedanz des Implantats so nachzuregeln, dass die daran angeschlossene Elektrode (ohne selbst per se MRI-sicher zu sein) den vorliegenden Umständen (wie Feldverteilungen) entsprechend stets hochfrequenztechnisch so abgeschlossen wird, dass die Erwärmung der Elektrodenspitze diesen Umständen entsprechend minimal ist.

Die Erfindung besteht somit zum einem in einem Sensor zur Temperaturmessung und zum anderen in einem Steuerverfahren zur wirkungsvollen Reduktion der MRT-induzierten Elektrodenerwärmung, wobei das Implantat diese Aufgabe erfüllen kann, und zwar weitestgehend unabhängig von der angeschlossenen Elektrodenleitung. Das so ausgestattete implantierbare System kann weitestgehend sicher im MRT eingesetzt werden.

In diesem Zusammenhang schafft der erfindungsgemäße Temperatursensor die Voraussetzung dafür, optional basierend auf einer Temperaturmessung durch adaptive Veränderungen der Implantatseigenschaften hinsichtlich der Eingangsimpedanz umstandsgemäß auch für Standardelektroden günstige Randbedingungen dafür zu schaffen, die Erwärmung der Elektrodenspitzen während Kernspintomographie (Magnetresonanztomographie, MRI) zu minimieren.

Ein implantierbares medizinisches System, dessen Bestandteil der erfindungsgemäße Temperatursensor sein kann, kann einen ein- oder mehrpolaren, temporär anwendbaren Katheter oder eine dauerimplantierbare Elektrodenleitung oder langgestrecktes elektrisch leitfähiges Implantat mit einer teilweisen Isolation aufweisen, bei denen jeweils an definierten Elektrodenflächen eine lokale Erwärmung, z.B. durch im MRT induzierte Ströme, zu erwarten ist. In einem solchen System kann der erfindungsgemäße Temperatursensor in einer der genannten Komponenten oder und einem an diese angeschlossenen Gerät (Implantat, Ablationsgenerator etc.) vorgesehen sein, wobei letzteres bevorzugt ist, um z.B. übliche Elektrodenleitungen verwenden zu können.

Ein solches System ermöglicht eine rein Implantat-seitige Lösung für das Erwärmungsproblem im MRT. Sowohl der für die Regelung erforderliche Temperatursensor wird mittels einer Standardelektrode ermöglicht und kann daher allein im Implantat realisiert werden als auch der zur Erwärmungsminimierung realisierte Mechanismus durch eine Stellvorrichtung kann durch eine entsprechend geregelte Implantatseingangsschaltung im Implantat realisiert werden.

Die Erfindung bietet eine Alternative zu solchen Ansätzen, die eine Spezialelektrode erfordern und kann daher zu einer nennenswerten Kostensenkung führen (Entwicklungs- und Herstellkosten). Zudem könnten bereits implantierte Elektroden als MRT-taugliche Systeme nur durch ein neues Implantat aufgerüstet werden, weil keine Elektrodenextraktion nötig ist, beliebige Elektrodenkombinationen mit dem Implantat verwendet werden können, etc.

Vorzugsweise ist der Temperatursensor ausgebildet, als das temperaturabhängige Merkmal der Impedanz deren Betrag, Phase, Realteil, Imaginärteil oder eine Kombination dieser Größen zu bestimmen, und zwar vorzugsweise für eine vorgegebene Frequenz oder mehrere Frequenzen. Der Temperatursensor kann auch ausgebildet sein, eine Kombination dieser Merkmale für mehrere Elektrodenpole gleichzeitig zu erfassen und auszuwerten.

Falls das implantierbare medizinische Gerät ein Herzstimulator ist, der zur Closed Loop Stimulation (CLS) mit einem entsprechenden Impedanzsenor ausgestattet ist, kann ein solcher CLS Sensor auch als Temperatursensor dienen. In solch einem Fall kann die im Implantat bereits vorhandene kontinuierliche Impedanzmessschaltung des CLS/HDS-Verfahrens für die Temperaturmessung genutzt werden. Die Closed Loop Stimulation und entsprechende Eigenschaften und Bestandteile hierfür ausgebildeter Herzstimulatoren sind in anderen Veröffentlichungen der Anmelderin näher beschrieben.

Bezüglich der Impedanzerfassung ergeben sich folgende vorteilhafte Ausführungsvarianten:
- Die Impedanz wird durch Einspeisung/Einprägung eines Stromes als Generatorsignal und Messung der resultierenden Spannung bestimmt.
- Die Impedanz wird durch Einspeisung/Einprägung einer Spannung und Messung des resultierenden Stromes bestimmt.
- Die Elektrodenpolimpedanz wird bevorzugt in einem Frequenzbereich von 0.1 Hz - 10 MHz gemessen.
- Die Impedanzerfassungseinrichtung ist ausgebildet, Messdaten nur in bestimmten Frequenzfenstern vor der Verarbeitung herauszufiltern. Ein bevorzugtes Frequenzintervall liegt hierfür zwischen 100kHz und 10MHz. Alle anderen Frequenzanteile des Messsignals werden vorzugsweise durch ein Bandpassfilter der Impedanzerfassungseinrichtung unterdrückt.
- Die Impedanzerfassungseinrichtung oder der Temperatursensor ist vorzugsweise ausgebildet, die Elektrodenpolimpedanzmessung bei ausgewählten Frequenzen durchzuführen, und zwar vorzugsweise jeweils diskret monofrequent, bei mindestens einer, bevorzugt mehrerer Frequenzen/Spektrallinien des Impedanzspektrums. Gemäß einer bevorzugten Ausführungsvariante ist die Impedanzerfassungseinrichtung ausgebildet, das Impedanzsignal engbandig um die jeweiligen einzelnen Messfrequenzen zu filtern, um potentielle Störungen besonders effektiv zu unterdrücken.
- Die Impedanzerfassungseinrichtung oder der Temperatursensor kann Filter aufweisen, um die Frequenz, Amplituden und Phasenlage des Generatorsignals zu erfassen, z.B. eine oder mehrere dementsprechende PLL Schaltungen.
- Die Impedanzerfassungseinrichtung kann auch ausgebildet sein, Messdaten nur in bestimmten Zeitfenstern vor der Verarbeitung herauszufiltern. Hierbei können die Zeitfenster durch MRI-Signale gesteuert sein. Das implantierbare medizinische Gerät kann hierfür einen MRT-Sensor aufweisen und nutzen, der ausgebildet ist, auf das Vorhandensein MRT-typischer Magnetfelder anzusprechen und ein Ausgangssignal zu erzeugen, das das Vorhandensein MRT-typischer Magnetfelder anzeigt. Alternativ oder zusätzlich können die Zeitfenster durch physiologische Signale gesteuert sein, d.h. die Impedanzmessung zur Temperaturbestimmung findet nur in bestimmen Herzzyklen oder Phasen eines jeweiligen Herzzyklus statt. Ziel ist es dadurch, die aufgrund temperaturbedingter Impedanzänderungen von solchen, die durch den Herzzyklus/Atemzyklus bedingt sind, zu trennen. Hierzu kann das implantierbare medizinische Gerät entsprechende, an sich bekannte Sensingeinheiten aufweisen.
- Vorzugsweise ist das Generatorsignal ein Rechtecksignal oder ein anderes oberwellenreiches Signal, wobei die Impedanzerfassungseinrichtung ausgebildet ist, eine Auswertung der Impedanzspektrallinien an den Harmonischen des Generatorsignals vorzunehmen.

Zur Trennung der temperaturbedingten von anders, z.B. physiologisch, bedingten Impedanzänderungen kann der Temperatursensor ausgebildet sein, auf einen ReferenzImpedanzverlauf zurückzugreifen, der in Zeiten aufgenommen und gespeichert wurde, wenn keine Kernspintomografie stattfindet. Der Temperatursensor kann insbesondere ausgebildet sein, regelmäßig neue Referenz-Impedanzverläufe aufzunehmen und zu diesem Zweck mit einem MRT-Sensor verbunden oder ausgestattet sein, der ausgebildet ist, auf das Vorhandensein MRT-typischer Magnetfelder anzusprechen und ein Ausgangssignal zu erzeugen, das das Vorhandensein MRT-typischer Magnetfelder anzeigt. Der Temperatursensor ist in diesem Fall ausgebildet, die Aufnahme und/oder Speicherung von Referenz-Impedanzverläufen zu unterbinden, solange der MRT-Sensor ein das Vorhandensein MRT-typischer Magnetfelder anzeigendes Ausgangssignal liefert.

Zur Trennung eines temperaturbedingen Impedanzverlaufs von durch andere als temperaturbedingte Ursachen bedingten Impedanzverläufen kann der Temperatursensor Morphologie-basierte und adaptive Filter aufweisen.

Gemäß einer bevorzugten Ausführungsvariante wirkt die Stellvorrichtung auf einen einstellbaren Kondensator, eine einstellbare Induktivität, einen einstellbaren Widerstand oder Kombination solcher Bauteile in der Eingangsschaltung oder Schutzschaltung des Implantats. Insbesondere können EMI Kondensatoren oder ein Teil derer (bzw. dazu in Reihe oder parallel geschaltet) als Varicap realisiert sein, Widerstände als FET-Transistor. Induktivitäten können durch aktive Bauelemente realisiert (z.B. Transformation einer Kapazität) sein. Vorzugsweise ist die Stellvorrichtung ausgebildet, eine spannungs- und/oder stromgesteuerte Abstimmung (Einstellung) dieser Elemente in Abhängigkeit des temperatur-bedingten Merkmals zu bewirken.

Die Stellvorrichtung kann mit einem Regler verbunden sein, dem das temperaturabhängige Merkmal als Sensorsignal zugeführt wird und der die Stellvorrichtung nachführt, um die Elektrodenpolerwärmung zu minimieren. Vorzugsweise verkörpert der Regler einen Regelalgorithmus basierend auf dem Gradientenverfahren, insbesondere für multidimensionale Regelung, d.h. Tuning einer Kombination mehrerer Elemente der Eingangsschaltung wie Kondensator, Induktivität und/oder Widerstand.

Der erfindungsgemäße Temperatursensor kann auch selbst als Teil eines MRT-Sensors oder als MRT-Sensor eingesetzt werden, d.h. das Ausgangssignal des Temperatursensors wird zur Festlegung der Entscheidung herangezogen, ob sich eine Elektrodenleitung oder ein Katheter mit einem oder mehreren Elektrodenpolen gerade im Wechselmagnetfeld eines Kernspintomografen befindet, d.h. eine bildgebende Sequenz läuft oder nicht. Eine Steuereinheit eines implantierbaren medizinischen Gerätes kann mit einem solchen MRT-Sensor verbunden und ausgebildet sein, das medizinische Gerät in einen speziellen MRIdesignierten Betriebsmodus zu schalten, falls der MRT-Sensor ein Ausgangssignal liefert, das das Vorhandensein MRT-typischer Magnetfelder anzeigt. Dies kann zusätzlich zu der Maßnahme, dass die Eingangsschaltung so parametriert wird, dass die Temperaturentwicklung am Elektrodenpol minimiert wird, geschehen.

Der Temperatursensor kann auch Teil einer Temperatursignalisierungseinrichtung im MRT sein, indem das Ausgangssignal des Temperatursensors dazu genutzt wird, um über eine drahtlose Schnittstelle dem MRT-Anwender eine übermäßige Elektrodenerwärmung anzuzeigen, beispielsweise falls die Abstimmung der Eingangsschaltung nicht ausreichend ist, die Erwärmung der Elektrodenpole hinreichend zu reduzieren. Die drahtlose Schnittstelle kann in Form einer Telemetrieeinheit realisiert sein, die dazu ausgebildet sein kann, Daten im MICS-Frequenzband an ein externes Gerät zu übertragen. Das MICS-Frequenzband ist ein Frequenzband, das für die Kommunikation mit medizinischen Implantaten reserviert ist (MICS: medical implant communication service).

Zur Lösung der vorgenannten Aufgabe trägt auch ein Verfahren zum Einstellen einer Eingangsimpedanz eines implantierbaren medizinischen Gerätes für den Anschluss einer Elektrodenleitung mit wenigstens einem längsgestreckten elektrischen Leiter und wenigstens einem mit dem Leiter verbundenen Elektrodenpol bei, welches die folgenden Verfahrensschritte aufweist:
- Erfassen einer temperaturbedingten Elektrodenpolimpedanzänderung und
- Einstellen der Eingangsimpedanz derart, dass die temperaturbedingte Elektrodenpolimpedanzänderung möglichst gering wird.

Weitere vorteilhafte Ausgestaltungen ergeben sich aus der Kombination der vorstehend beschriebenen Merkmale und aus den nachfolgend beschriebenen Ausführungsbeispielen.

Die Erfindung soll nun anhand von Ausführungsbeispielen mit Bezug auf die Figuren näher erläutert werden. Die Figuren zeigen Folgendes:
- Fig. 1: zeigt als implantierbare medizinische Geräte einen implantierbaren Herzstimulator 10 und eine daran angeschlossene implantierbare Elektrodenleitung 20.
- Fig. 2: zeigt beispielhaft einige Komponenten eines erfindungsgemäßen implantierbaren medizinischen Gerätes, wie es beispielsweise in Figur 1 dargestellt ist.
- Fig. 3: zeigt die Temperaturabhängigkeit der Elektrodenpolimpedanz.
- Fig. 4: zeigt ein CLS-Nutzsignal gegenüber einem CLS-Temperatursignal.
- Fig. 5: zeigt ein vereinfachtes Blockschaltbild einer ersten Ausführungsvariante für die erfindungsgemäße Impedanzauswertungseinheit.
- Fig. 6: zeigt ein vereinfachtes Blockschaltbild einer zweiten Ausführungsvariante für die erfindungsgemäße Impedanzauswertungseinheit mit alternativen oder zusätzlichen Schaltern in Reihe zu den Elektrodenleitungen.
- Fig. 7: zeigt ein geeignetes Generatorsignal für die Impedanzmessung.

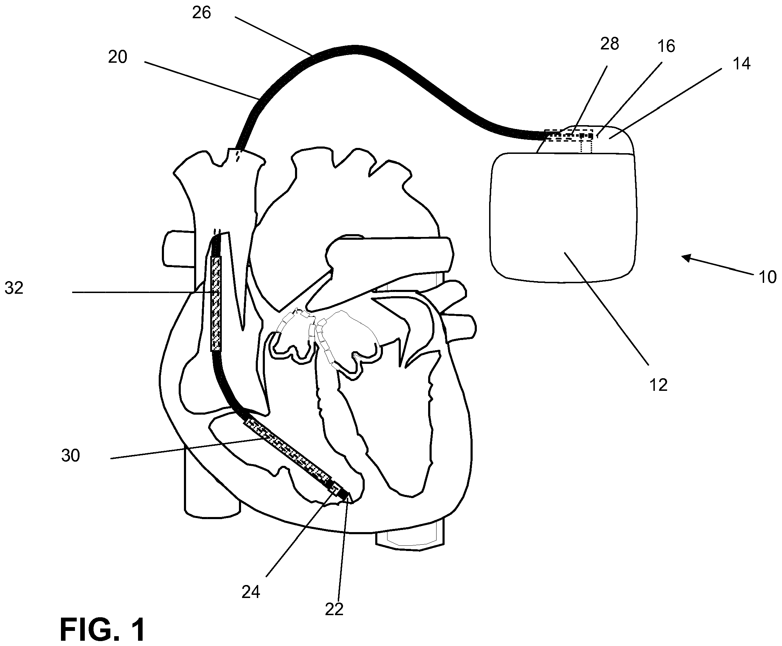

In Figur 1 ist ein implantierbares medizinisches Gerät in Form eines implantierbaren Herzstimulators 10 dargestellt, an den eine Elektrodenleitung 20, die einen langgestreckten Leiter aufweist, angeschlossen ist.

Der implantierbare Herzstimulator 10 kann ein Herzschrittmacher oder ein Kardioverter/Defibrillator (ICD) sein. In dem in Figur 1 dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

Die Elektrodenleitung 20 stellt im Sinne dieser Erfindung ein implantierbares medizinisches Gerät mit einem langgestreckten elektrischen Funktionsleiter dar. An einem distalen Ende der Elektrodenleitung 20 sind in an sich bekannter Manier Elektrodenpole in Form einer Spitzen- oder Tipelektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen, an das sie umgebende Herzgewebe, ausgebildet sind. Fig. 1 zeigt, wie sich die Elektrodenpole, also die Tipelektrode 22 und die Ringelektrode 24, im Anwendungsfall die Elektrodenleitung 20, im Apex eines rechten Ventrikels eines Herzens befinden.

Sowohl die Tipelektrode 22 als auch die Ringelektrode 24 sind über jeweils wenigstens einen elektrischen Leiter 26 mit einem Kontakt eines Steckers 28 am proximalen Ende der Elektrodenleitung 20 elektrisch verbunden. Der Stecker 28 besitzt elektrische Kontakte, die mit den elektrischen Kontakten 16 der Kontaktbuchse im Anschlussgehäuse 14 des implantierbaren Herzstimulators korrespondieren.

Die elektrischen Leiter 26 in der Elektrodenleitung 20 können als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden, dienen dazu, elektrische Signale vom Steckkontakt zum jeweiligen Elektrodenpol zu übertragen oder abgefühlte elektrische Potenziale repräsentierende Signale vom jeweiligen Elektrodenpol zum Steckkontakt zu führen.

Die elektrischen Leiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blankliegenden, helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann aber jedes andere an sich bekannte elektromedizinische Implantat, also auch ein Mehrkammer-Herzschrittmacher oder Kardioverter/Defibrillator (ICD) oder auch ein Neurostimulator oder ein reines Monitoring-Implantat, dienen. Auch die Anzahl der Elektrodenleitungen, die vom Implantat zu dem oder den Stimulationsorten und/oder Abfühlorten führen kann variieren.

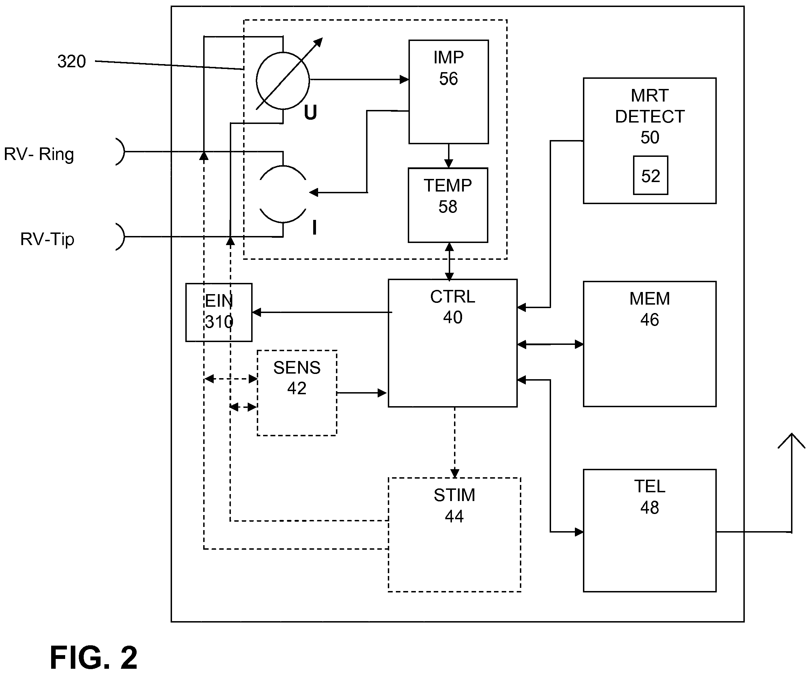

Figur 2 zeigt beispielhaft und schematisch einige Komponenten des Herzstimulators 10 aus Figur 1. Typische Komponenten eines solchen Herzstimulators sind eine Steuereinheit 40, eine oder mehrere Sensingeinheiten 42, die jeweils eine Diagnoseeinheit darstellen und eine oder mehrere Stimulationseinheiten 44, die jeweils eine Therapieeinheit darstellen. Die Steuereinheit 40 ist zuvor mit der Sensingeinheit 42 als auch mit der Stimulationseinheit 44 verbunden. Sowohl die Sensingeinheit 42 als auch die Stimulationseinheit 44 sind jeweils mit Elektrodenanschlüssen verbunden, um im Falle der Sensingeinheit 42 elektrische Potenziale des Herzgewebes über die rechtsventrikuläre Ringelektrode 24 und/oder die rechtsventrikuläre Tipelektrode 22 erfassen zu können bzw. im Falle der Stimulationseinheit 44 Stimulationsimpulse beispielsweise über die rechtsventrikuläre Ringelektrode 24 und/oder die rechtsventrikuläre Tipelektrode 22 ausgeben zu können.

Außerdem ist die Steuereinheit 40 mit einer Speichereinheit 46 zum Speichern erfasster Werte von jeweils zu messenden Parametern verbunden. Eine ebenfalls mit der Steuereinheit 40 verbundene Telemetrieeinheit 48 erlaubt es, erfasste Werte von Parametern an ein externes Gerät zu übertragen oder Steuerbefehle von einem externen Gerät zu empfangen.

Die Steuereinheit 40 ist außerdem mit einem MRT-Sensor 50 verbunden, der dazu ausgebildet ist, MRT-typische Magnetfelder zu erkennen und ein Ausgangssignal an die Steuereinheit 40 auszugeben, welches das Vorhandensein solcher MRT-typischer Magnetfelder anzeigt. Der MRT-Sensor 50 besitzt hierzu einen Magnetfeldsensor 52.

Alternativ kann der MRT-Sensor auch so ausgebildet sein, dass er von einem gepulsten elektromagnetischen Wechselfeld des MRT herrührende, in eine Programmierspule (nicht gezeigt) des Herzstimulators 10 induzierte Spannungen oder Spannungsverläufe erkennt und ein MRT-Erkennungssignal an die Steuereinheit 40 übermittelt. Der MRT-Sensor kann dann ausgebildet sein, auf ein Feldstärke-abhängiges Sättigungsverhalten des Ferittkerns anzusprechen.

Auch kann der MRT-Sensor 50 mit einem Transformator mit Ferittkern (nicht dargestellt) verbunden sein, der seinerseits mit einem länglichen elektrischen Leiter, z.B. der Elektrodenleitung, verbunden ist.

Die Steuereinheit 40 ist außerdem mit einer Impedanzbestimmungseinheit 56 verbunden. Die Impedanzbestimmungseinheit 56 ist mit einer Stromquelle I und einer Spannungsmesseinheit U verbunden, die ihrerseits wiederum mit den Anschlüssen für die Ringelektrode 24 und die Tipelektrode 22 verbunden sind. Auf diese Weise kann die Gleichstromquelle I konstant Stromimpulse über die Tipelektrode 22 und die Ringelektrode 24 abgeben und die Spannungsmesseinheit U kann die dabei jeweils abfallende Spannung messen. Aus diesen Werten kann die Impedanzbestimmungseinheit 56 einen jeweiligen Impedanzwert bestimmen. Die Impedanzbestimmungseinheit 56 bildet zusammen mit der Gleichstromquelle I und der Spannungsmesseinheit U eine Impedanzerfassungseinheit.

Ein von der Impedanzbestimmungseinheit 56 bestimmter Impedanzwert hängt von verschiedenen Einflussgrößen ab. Beispielsweise würde sich ein Bruch eines elektrischen Leiters in der Elektrodenleitung 20 in einem sehr hohen Impedanzwert äußern. Bei intakter Elektrodenleitung 20 hängt die zwischen den Elektrodenpolen 22 und 24 zu messende Impedanz auch von der Blutmenge im rechten Ventrikel eines Herzens ab, so dass die zu messende Impedanz entsprechend dem Herzzyklus zyklisch schwankt. Beispielsweise steigt die Impedanz mit abnehmendem Blutvolumen, d. h. mit abnehmendem Volumen der rechten Herzkammer, so dass ein zyklischer Anstieg der Impedanz die zyklische Kontraktion der rechten Herzkammer (des rechten Ventrikels) anzeigt. Ebenso kann ein entsprechender Anstieg der gemessenen Impedanz infolge einer Kammerkontraktion nach Abgabe eines Stimulationsimpulses den Stimulationserfolg anzeigen. Auf diese Weise ist die Impedanzbestimmungseinheit 56 in der Lage, eine automatische Stimulationserfolgskontrolle (automatic capture control; ACC) durchzuführen.

Die gemessene Impedanz hängt außerdem von der Impedanz des Elektrodenpol-Gewebekontaktes ab. Daher lässt sich durch Auswerten der gemessenen Impedanzwerte auch eine Ödembildung erfassen, wie sie beispielsweise durch eine Erwärmung der Elektrodenpole infolge magnetischer Wechselfelder eines Kernspintomografen auftreten kann.

Für die vorliegende Erfindung ist es von Bedeutung, dass die Impedanz auch von der Temperatur der Elektroden und der die Elektroden umgebenden Flüssigkeit abhängt. Die Leitfähigkeit (sigma) der Flüssigkeit ist temperaturabhängig; für den hier angenommenen wasserbasierten Elektrolyten geht man von einem Temperaturkoeffizient von ca. 2%/K aus. Dies kann für die erwarteten Erwärmungswerte zu Leitfähigkeitsvariationen von gut mehr als 20% führen.

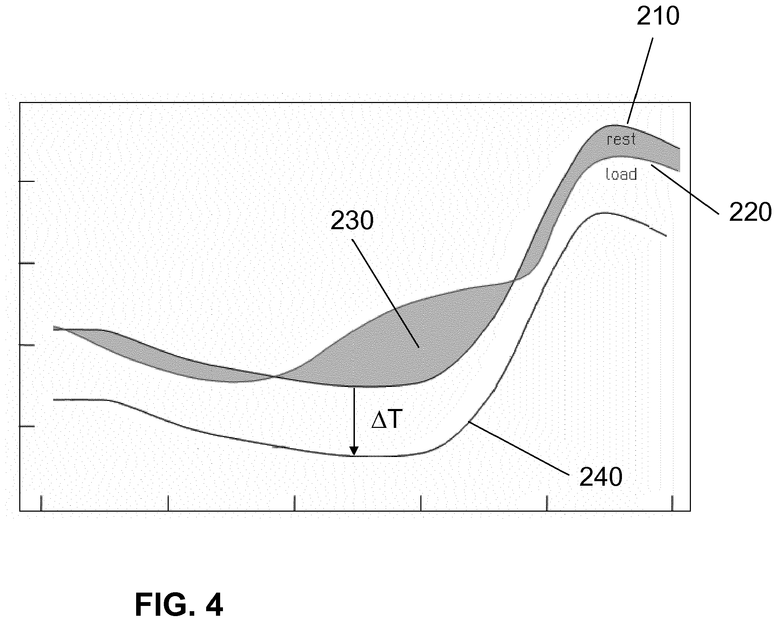

Dieser Effekt wird von einem Temperatursensor 58 genutzt, der mit der Impedanzbestimmungseinheit 56 verbunden ist und das Impedanzsignal, wie einleitend beschrieben und im Zusammenhang mit Figur 4 näher erläutert, auswertet, um ein eine Elektrodenpoltemperatur anzeigendes Temperatursignal an die Steuereinheit 40 zu liefern.

Es sei darauf hingewiesen, dass die Impedanzbestimmungseinheit 56 nicht nur indirekt, wie in Figur 2 dargestellt, sondern zusätzlich auch direkt und/oder über eine CLS-Auswerteeinheit mit der Steuereinheit 40 verbunden sein kann.

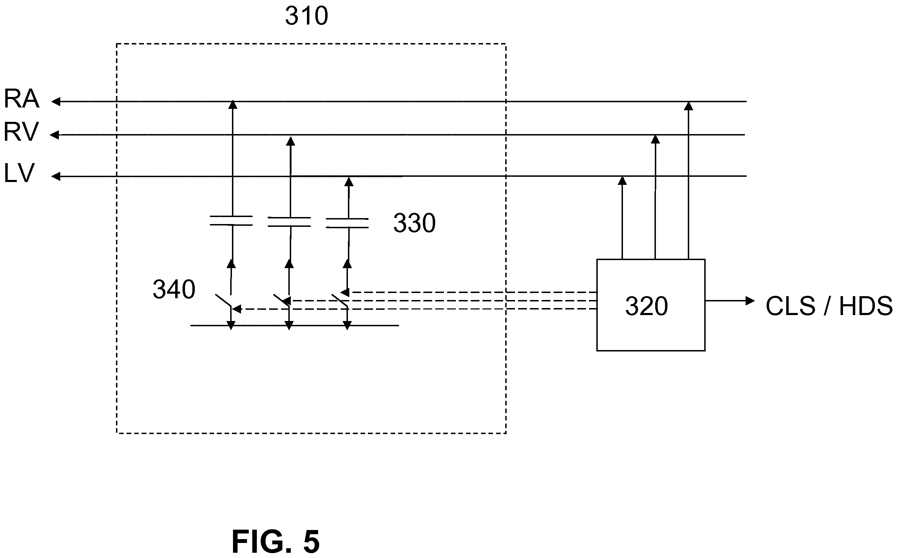

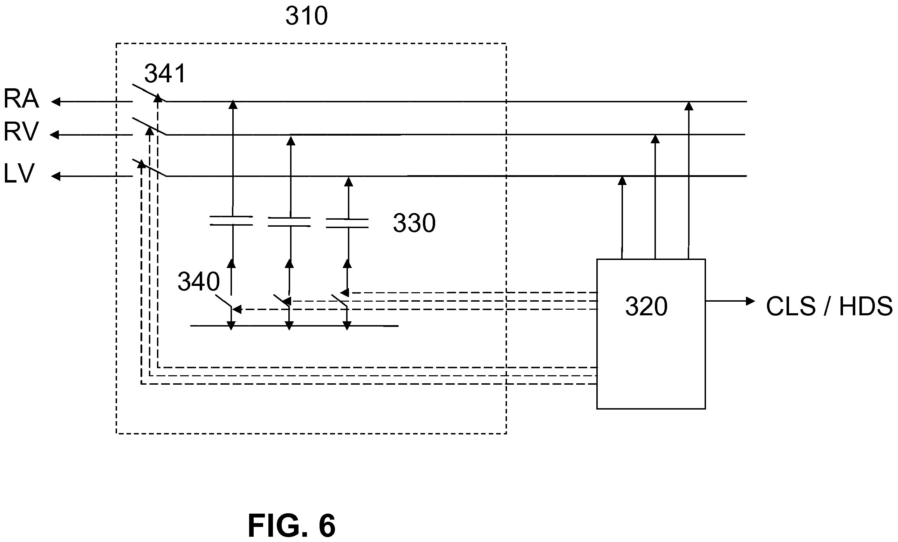

Die Steuereinheit ist unter anderem ausgebildet, aus dem Temperatursignal ein Steuersignal für eine Eingangsstufe 320 des Implantats 10 zu bilden. Die Eingangsstufe 320 enthält eine Eingangsschaltung, die unter anderem die für die angeschlossenen Elektrodenleitungen relevante Eingangsimpedanz des Implantats 10 bestimmt. Die Eingangsimpedanz ist einstellbar, wie einleitend und weiter unten mit Bezug auf Figuren 5 und 6 näher erläutert ist.

Die Steuereinheit realisiert in Bezug auf die Eingangsimpedanz der Eingangsstufe 320 einen Regler, der von dem Temperatursignal gesteuert und dazu ausgelegt ist, die Eingangsimpedanz möglichst so einzustellen, dass die temperatur-bedingte Impedanzänderung möglichst klein ist, d.h. dass die Elektrodenleitung durch Einstellung der Eingangsimpedanz so abgeschlossen wird, dass die Erwärmung der Elektrodenpole möglichst gering ist.

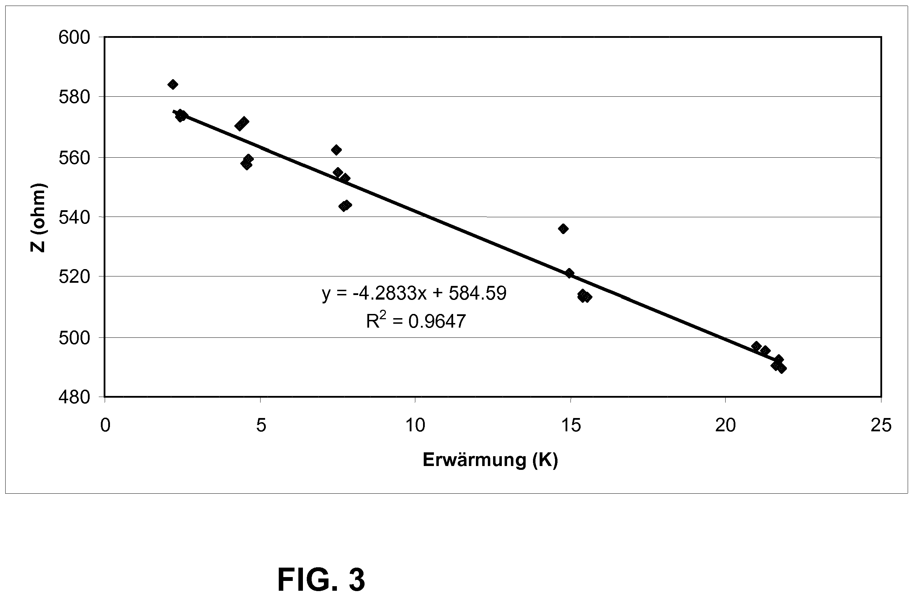

Ein Beispiel für die Temperaturabhängigkeit der Elektrodenpolimpedanz und Nachweis der Wirksamkeit ist in Figur 3 gezeigt. Die Messungen wurden mit sinusförmigen Generatorsignalen bei 10 kHz gemacht. Das Messmedium ist konvektionshemmendes Gel nach Standard ASTM F2182.

Es wird erwartet, dass eine Temperaturerhöhung von 15-20K für wenige Sekunden ohne permanente Schadensfolgen tolerabel ist. In dieser Zeit ist ein ausreichender Signalhub zu erzielen, dass der Regler zuverlässig und fehlertolerant arbeiten kann.

In der Figur 4 ist im Anwendungsbeispiel CLS der Unterschied zwischen dem aus den CLS-Signalen ermittelten Kontraktilitätswert und dem Temperaturwert dargestellt. Die Impedanzkurve 210 zeigt beispielhaft den Verlauf der unipolaren intrakardialen Impedanz an der Elektrodenspitze (Tip) in Ruhe und die Kurve 220 den gleichen Impedanzverlauf bei Belastung. Zur Auswertung für die CLS- bzw. Kontraktilitätsanalyse wird bekanntermaßen die Flächendifferenz 230 beider Kurven ausgewertet und so ein Maß für die Kontraktilität/metabolische Anforderung bestimmt, das beispielsweise zur Stimulationsratenanpassung genutzt werden kann.

Bei der erfindungsgemäßen Anwendung der Impedanzmessfunktion ist zur Temperaturmessung nun zusätzlich eine Auswertung des absoluten Offset der Messkurven erforderlich. In der Kurve 240 ist die zu erwartende Impedanzkurve in Ruhe bei gesteigerter Elektrodenspitzentemperatur gezeigt. Als Maß für die Temperatur wird nun erfindungsgemäß in einer Offsetbestimmungseinheit der Offset der Impedanzkurve bezogen auf einen Referenzkurve ausgewertet und daraus eine Korrelation zum Temperaturanstieg (ΔT) gemäß Figur 1 und Figur 3 hergestellt. Als Referenzkurven (Refernzverläufe) kommen dabei prinzipiell die auch für CLS aufgezeichneten Impedanzreferenzkurven, abhängig von der aktuellen Eventfolge (Ax-Vx) in Frage, allerdings werden für die erfindungsgemäße Anwendung andere (kürzere) Zeitkonstanten für die Aktualisierung der Referenzkurven angenommen.

In der Figur 5 ist ein einfaches Blockschaltbild für die erfindungsgemäße Vorrichtung dargestellt. Die Elektrodenleitungen (hier für einen Drei-Kammer-Herzstimulator mit rechts-atrialer Elektrodenleitung RA, rechtsventrikulärer Elektrodenleitung RV und linksventrikulärer Elektrodenleitung LV) sind in der üblichen Weise mit einer Eingangsstufe 310 des Implantates 10 verbunden. Die Eingangsstufe 310 bildet wenigstens einen Teil einer Eingangsschaltung des Herzstimulators 10. Innerhalb des Implantates sind eine oder alle Elektrodenleitungen wiederum mit einer erfindungsgemäß erweiterten Impedanz- und Temperaturauswerteeinheit 320 verbunden, die die Impedanzmesseinheit 56 und den Temperatursensor 58 aufweist und die eine kontinuierliche Impedanzmessung der jeweiligen Elektroden durchführt. Diese Signale werden optional auch für die CLS- und/oder HDS- und/oder LES-Auswertungen (Lung fluide sensor) im Implantat 10 durch dessen Steuereinheit 40 weiterverwendet.

Erfindungsgemäß wertet die Impedanzmessstufe 320 die Impedanzkurven zusätzlich gemäß Figur 4 aus, so dass eine Steuerinformation abhängig von der jeweiligen Elektrodentemperatur gebildet werden kann. Diese Steuerinformation veranlasst dann die Impedanzmesseinheit, die Charakteristik des Eingangsstufenanpassungsnetzwerkes 330 über eine zusätzliche Schaltvorrichtung 340 derart zu variieren, dass eine Reduktion der Elektrodenpoltemperatur infolge einer Verstimmung der jeweiligen "Elektrodenantennencharakteristik" herbeigeführt wird. Dieses Regelprinzip funktioniert dabei unabhängig vom Typ der eingesetzten Elektrodenleitung. Das Eingangsstufenanpassungsnetzwerk 330 umfasst mehrere Kondensatoren, die mittels der Schalter 340 abgestimmt oder zu- bzw. abgeschaltet werden können, so dass die Eingangskapazität und damit die Eingangsimpedanz der Eingangsstufe 310 des Herzstimulators 10 entsprechend anpassbar ist. Die Schaltvorrichtung 340 bildet somit eine Stellvorrichtung für die Eingangsimpedanz der Eingangsstufe 310.

Gemäß einer weiteren in Figur 6 abgebildeten Ausführungsvariante sind alternativ oder zusätzlich Schalter in Reihe zu den Elektrodenleitungen eingebaut, durch die ebenso die Abschlussimpedanz der Elektrodenleitungen zu- oder weggeschaltet werden kann. In einer besonderen Ausführung, insbesondere bei ICDs, werden diese Schalter durch die schon vorhandenen Protektion Transistoren realisiert, bevorzugt sind diese dann einzeln ansteuerbar.

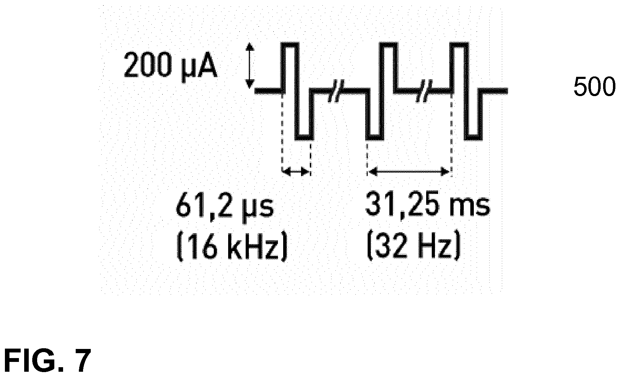

Die Figur 7 zeigt ein übliches Generatorsignal 500 zur Impedanzmessung. Dieses ist typischerweise eine Konstantstrompulsfolge mit einer Amplitude und Pulsbreite deutlich unterhalb der Reizschwelle des mit dem Generatorsignal beaufschlagten Gewebes.

## Patentansprüche

1. Temperatursensor (58) zum Erfassen einer Erwärmung wenigstens eines Elektrodenpols (22, 24) einer temporär oder dauerhaft implantierbaren Elektrodenleitung (20) oder eines ähnlichen Implantats mit wenigstens einem langgestreckten elektrischen Leiter, der mit wenigstens einem Elektrodenpol (22, 24) verbunden ist,
wobei der Temperatursensor (58) eine Impedanzerfassungseinrichtung (56) aufweist oder mit einer solchen verbunden ist, wobei die Impedanzerfassungseinrichtung (56) mit dem wenigstens einen Elektrodenpol (22, 24) elektrisch verbunden ist, und wobei der Temperatursensor (58) dazu ausgelegt ist, zum Erfassen der Erwärmung des wenigstens einen Elektrodenpols (22, 24) eine von der Impedanzerfassungseinrichtung (56) erfasste Elektrodenpolimpedanz auszuwerten,
wobei die Auswertung hinsichtlich eines temperaturabhängigen Merkmals der Elektrodenpolimpedanz erfolgt,
**dadurch gekennzeichnet, dass** der Temperatursensor (58) oder die Impedanzerfassungseinrichtung (56) ausgebildet ist, eine Elektrodenpolimpedanz nur innerhalb wenigstens eines ereignisabhängigen Zeitfensters zu erfassen oder auszuwerten.

2. Temperatursensor (58) nach Anspruch 1, **dadurch gekennzeichnet, dass** der Temperatursensor (58) ausgebildet ist, als das temperaturabhängige Merkmal der Impedanz deren Betrag, Phase, Realteil, Imaginärteil oder eine Kombination dieser Werte zu bestimmen.

3. Temperatursensor (58) nach einem der Ansprüche 1 oder 2, **dadurch gekennzeichnet, dass** der Temperatursensor (58) oder die Impedanzerfassungseinrichtung (56) ausgebildet ist, eine Elektrodenpolimpedanz nur innerhalb eines oder mehrerer vorgegebener Frequenzbereiche auszuwerten.

4. Temperatursensor (58) nach einem der Ansprüche 1 bis 3, **dadurch gekennzeichnet, dass** die Impedanzerfassungseinrichtung (56) oder der Temperatursensor (58) Filter aufweisen, um die Frequenz, Amplitude und Phasenlage eines Generatorsignals zu erfassen.

5. Temperatursensor (58) nach einem der vorigen Ansprüche, **dadurch gekennzeichnet, dass** das Zeitfenster von einem innerhalb aufeinander folgender Herzzyklen wiederkehrenden Signalmerkmal oder Ereignis gesteuert ist.

6. Temperatursensor (58) nach einem der vorigen Ansprüche, **dadurch gekennzeichnet, dass** das Zeitfenster von einem MRT-Sensor gesteuert ist, der ausgebildet ist, auf das Vorhandensein MRT-typischer Magnetfelder anzusprechen und ein Ausgangssignal zu erzeugen, das das Vorhandensein MRT-typischer Magnetfelder anzeigt.

7. Temperatursensor (58) nach einem der vorigen Ansprüche, **dadurch gekennzeichnet, dass** der Temperatursensor (58) zur Trennung eines temperaturbedingen Impedanzverlaufs von durch andere als temperaturbedingte Ursachen bedingten Impedanzverläufen ausgebildet ist.

8. Temperatursensor (58) nach Anspruch 7, **dadurch gekennzeichnet, dass** der Temperatursensor (58) zur Trennung eines temperaturbedingen Impedanzverlaufs von durch andere als temperaturbedingte Ursachen bedingten Impedanzverläufen auf einen Referenzimpedanzverlauf zurückgreift.

9. Implantierbares medizinisches Gerät (10) mit Temperatursensor (58) nach einem der Ansprüche 2 bis 8, wobei an das implantierbare medizinische Gerät wenigstens eine Elektrodenleitung (20) mit wenigstens einem Elektrodenpol (22, 24) anschließbar oder angeschlossen ist.

10. Implantierbares medizinisches Gerät (10) mit Temperatursensor (58) nach einem der Ansprüche 1 bis 8, wobei das implantierbare medizinische Gerät mit einer Stellvorrichtung ausgerüstet ist, die in Abhängigkeit des temperaturabhängigen Merkmals einer Elektrodenpolimpedanz eine Implantatseingangsschaltung hinsichtlich der Elektrodenabschlussimpedanz so parametriert, dass eine Erwärmung an dem jeweiligen Elektrodenpol (22, 24) minimiert wird, wobei die Stellvorrichtung auf einen einstellbaren Kondensator, eine einstellbare Induktivität, einen einstellbaren Widerstand oder Kombination solcher Bauteile in der Eingangsschaltung des implantierbaren medizinischen Gerätes wirkt.

11. Implantierbares medizinisches Gerät (10) nach Anspruch 9 oder 10, wobei die Stellvorrichtung mit einem Regler verbunden ist, dem das temperaturabhängige Merkmal als Sensorsignal zugeführt wird und der die Stellvorrichtung nachführt, um die Elektrodenpolerwärmung zu minimieren.

12. Verfahren zum Einstellen einer Eingangsimpedanz eines implantierbaren medizinischen Gerätes für den Anschluss einer Elektrodenleitung (20) mit wenigstens einem längsgestreckten elektrischem Leiter und wenigstens einem mit dem Leiter verbundenen Elektrodenpol (22, 24), **gekennzeichnet durch** die Schritte:
- Erfassen einer temperaturbedingten Elektrodenpolimpedanzänderung und
- Einstellen der Eingangsimpedanz derart, dass die an die anschließbare Elektrodenleitung (20) angeschlossene Elektrode hochfrequenztechnisch so abgeschlossen wird, dass die Erwärmung der Elektrodenspitze möglichst gering ist.

## Claims

1. A temperature sensor (58) for detecting heating of at least one electrode pole (22, 24) of a temporarily or permanently implantable electrode line (20) or a similar implant having at least one elongate electrical conductor which is connected to at least one electrode pole (22, 24),
wherein the temperature sensor (58) comprises an impedance detection unit (56) or is connected to such a unit,
wherein the impedance detection unit (56) is electrically connected to the at least one electrode pole (22, 24),
and wherein the temperature sensor (58) is designed, in order to detect the heating of the at least one electrode pole (22, 24), to evaluate an electrode pole impedance detected by the impedance detection unit (56),
wherein the evaluation is performed in respect of a temperature-dependent feature of the electrode pole impedance,
**characterised in that** the temperature sensor (58) or the impedance detection unit (56) is configured to detect or to evaluate an electrode pole impedance only within at least one event-dependent time window.

2. The temperature sensor (58) according to claim 1, **characterised in that** the temperature sensor (58) is designed to determine an amount, phase, real part, imaginary part of impedance or a combination of these values as the temperature-dependent feature of the impedance.

3. The temperature sensor (58) according to one of claims 1 or 2, **characterised in that** the temperature sensor (58) or the impedance detection unit (56) is configured to evaluate an electrode pole impedance only within one or more predefined frequency ranges.

4. The temperature sensor (58) according to any one of claims 1 to 3, **characterised in that** the impedance detection unit (56) or the temperature sensor (58) comprises filters in order to detect the frequency, amplitude and phase position of a generator signal.

5. The temperature sensor (58) according to any one of the preceding claims, **characterised in that** the time window is controlled by a signal feature or event reoccurring within successive heart cycles.

6. The temperature sensor (58) according to any one of the preceding claims, **characterised in that** the time window is controlled by an MRI sensor which is configured to respond to the presence of MRI-typical magnetic fields and to generate an output signal which indicates the presence of the MRI-typical magnetic fields.

7. The temperature sensor (58) according to any one of the preceding claims, **characterised in that** the temperature sensor (58) is configured to separate a temperature-related impedance curve from impedance curves based on causes other than temperature-related causes.

8. The temperature sensor (58) according to claim 7, **characterised in that** the temperature sensor (58) refers to a reference impedance curve in order to separate a temperature-related impedance curve from impedance curves based on causes other than temperature-related causes.

9. An implantable medical apparatus (10) with temperature sensor (58) according to any one of claims 2 to 8, wherein at least one electrode line (20) having at least one electrode pole (22, 24) is connectable or connected to the implantable medical apparatus.

10. The implantable medical apparatus (10) with temperature sensor (58) according to any one of claims 1 to 8, wherein the implantable medical apparatus is equipped with an adjusting device that is configured to parameterise an implant input circuit with respect to the electrode terminating impedance depending on the temperature-dependent feature of an electrode pole impedance in such a manner that heating at the electrode pole in question (22, 24) is minimised, wherein the adjusting device acts on an adjustable capacitor, an adjustable inductor, an adjustable resistor or combination of such components in the input circuit of the implantable medical apparatus.

11. The implantable medical apparatus (10) according to claim 9 or 10, wherein the adjusting device is connected to a controller to which the temperature-dependent feature is fed as a sensor signal and which readjusts the adjusting device to minimise electrode pole heating.

12. A method for setting an input impedance of an implantable medical apparatus for the connection of an electrode line (20) having at least one elongate electrical conductor and at least one electrode pole (22, 24) connected to the conductor, **characterised by** the steps:
- detecting a temperature-dependent change of electrode pole impedance; and
- setting the input impedance in such a way that the electrode connected to the connectable electrode line (20) is terminated in terms of high-frequency such that the heating of the electrode tip is minimal.

## Revendications

1. Capteur de température (58) pour la détection d'un réchauffement d'au moins un pôle d'électrode (22, 24) d'une ligne d'électrode (20) implantable de manière temporaire ou permanente ou d'un implant similaire avec au moins un conducteur électrique étiré en longueur qui est relié avec au moins un pôle d'électrode (22, 24), où le capteur de température (58) présente un dispositif de détection d'impédance (56) ou est relié avec un tel dispositif,
où le dispositif de détection d'impédance (56) est relié électriquement avec l'au moins un pôle d'électrode (22, 24),
et où le capteur de température (58) est conçu pour, en vue de la détection du réchauffement de l'au moins un pôle d'électrode (22, 24), évaluer une impédance de pôle d'électrode détectée par le dispositif de détection d'impédance (56),
où l'évaluation a lieu en ce qui concerne une caractéristique de l'impédance de pôle d'électrode dépendante de la température,
**caractérisé en ce que** le capteur de température (58) ou le dispositif de détection d'impédance (56) sont conçus pour détecter ou évaluer une impédance de pôle d'électrode uniquement dans le cadre d'au moins une fenêtre temporelle dépendant d'un événement.

2. Capteur de température (58) selon la revendication 1, **caractérisé en ce que** le capteur de température (58) est conçu pour déterminer, en tant que caractéristique dépendante de la température de l'impédance, sa contribution, sa phase, sa composante réelle, sa composante imaginaire ou une combinaison de ces valeurs.

3. Capteur de température (58) selon l'une des revendications 1 ou 2, **caractérisé en ce que** le capteur de température (58) ou le dispositif de détection d'impédance (56) sont conçus pour évaluer une impédance de pôle d'électrode uniquement dans une ou plusieurs plages de fréquences prédéfinies.

4. Capteur de température (58) selon l'une des revendications 1 à 3, **caractérisé en ce que** le dispositif de détection d'impédance (56) ou le capteur de température (58) présentent des filtres afin de détecter la fréquence, l'amplitude et la position de phase d'un signal de générateur.

5. Capteur de température (58) selon l'une des revendications précédentes, **caractérisé en ce que** la fenêtre temporelle est commandée par une caractéristique de signal ou un événement se répétant dans des cycles cardiaques se succédant les uns aux autres.

6. Capteur de température (58) selon l'une des revendications précédentes, **caractérisé en ce que** la fenêtre temporelle est commandée par un capteur d'imagerie par résonance magnétique MRT qui est conçu pour s'appliquer à la présence de champs magnétiques typiques en MRT et pour générer un signal de sortie qui indique la présence de champs magnétiques typiques en MRT.

7. Capteur de température (58) selon l'une des revendications précédentes, **caractérisé en ce que** le capteur de température (58) est conçu pour la séparation d'une évolution d'impédance liée à la température parmi des évolutions d'impédance reliées à d'autres causes que celles reliées à la température.

8. Capteur de température (58) selon la revendication 7, **caractérisé en ce que** le capteur de température (58) a recours à une évolution d'une impédance de référence pour la séparation d'une évolution d'impédance liée à la température parmi des évolutions d'impédance reliées à d'autres causes que celles reliées à la température.

9. Appareil médical implantable (10) doté d'un capteur de température (58) selon l'une des revendications 2 à 8, dans lequel au moins une ligne d'électrode (20) peut être connectée ou est connectée à l'appareil médical implantable avec au moins un pôle d'électrode (22, 24).

10. Appareil médical implantable (10) doté d'un capteur de température (58) selon l'une des revendications 1 à 8, où l'appareil médical implantable est équipé d'un dispositif de réglage qui paramètre, en fonction de la caractéristique dépendante de la température d'une impédance de pôle d'électrode, un circuit d'entrée d'implant en ce qui concerne l'impédance de terminaison d'électrode, de telle manière qu'un réchauffement au niveau du pôle d'électrode (22, 24) respectif est minimisé, où le dispositif de réglage agit au niveau d'un condensateur réglable, d'une inductance réglable, d'une résistance réglable ou d'une combinaison de tels composants dans le circuit d'entrée de l'appareil médical implantable.

11. Appareil médical implantable (10) selon la revendication 9 ou la revendication 10, dans lequel le dispositif de réglage est relié avec un régleur vers lequel la caractéristique dépendante de la température est menée sous forme d'un signal de capteur et qui actualise le dispositif de réglage afin de minimiser l'échauffement du pôle d'électrode.

12. Procédé de réglage d'une impédance d'entrée d'un appareil médical implantable permettant la connexion d'une ligne d'électrode (20) à au moins un conducteur électrique étiré en longueur et au moins un pôle d'électrode (22, 24) relié au conducteur, **caractérisé par** les étapes :
- de détection d'une modification d'impédance de pôle d'électrode dépendante de la température, et
- de réglage d'une impédance d'entrée de telle manière que l'électrode connectée à la ligne d'électrode (20) raccordable peut être verrouillée en haute fréquence de sorte que l'échauffement de la pointe d'électrode reste le plus faible possible.
